# EUROPEAN PATENT APPLICATION

(11) **EP 4 745 982 A1**
(43) Date of publication of application: **20.05.2026**
(21) Application number: 25209980.9
(22) Date of filing: 21.10.2025
(51) Int. Cl.: G16H 30/20, G16H 50/20, G16H 50/30, A61B 6/50, A61B 6/02, G06T 7/00

(54) **SMART PROTOCOLS FOR EFFICIENT TOMOSYNTHESIS DATA REVIEW**

(30) Priority: 15.11.2024 US 202418949683
(71) Applicant: GE Precision Healthcare LLC, Waukesha, WI 53188 (US)
(72) Inventor: BERNARD, Sylvain, 78533 Buc (FR); BISMUTH, Vincent Jonas, 78533 Buc (FR); GUNES, Gokhan, 78533 Buc (FR); IORDACHE, Razvan, 78533 Buc (FR); PEYRONNET, Jean Marc, 78533 Buc (FR)
(74) Representative: Kilburn & Strode LLP

(57) **Abstract**

Various methods and systems are provided for reducing an amount of time and resources spent on reviewing digital breast tomosynthesis (DBT) images and corresponding synthetic 2D images. In one example, a method includes receiving a plurality of tomosynthesis data studies, each study comprising a first image set of synthetic two-dimensional (S2D) images, a second image set of slab images, and a third image set of plane images. For each study of the plurality of tomosynthesis data studies, a minimum dataset to be reviewed by a radiologist is determined based on an analysis of the tomosynthesis data by an AI-based system, and the minimum dataset is displayed on a display device rather than the plurality of tomosynthesis data studies.

## Description

### FIELD

Embodiments of the subject matter disclosed herein relate to digital mammography imaging procedures.

### BACKGROUND

Mammography makes it possible to obtain two-dimensional (2D) images of a subject's breast. Digital breast tomosynthesis (DBT) makes it possible to obtain a three-dimensional (3D) image volume of the subject's breast, which is achieved by 3D reconstruction of the breast from a plurality of 2D projections of an area of the breast along distinct directions. The 3D modelling is typically carried out by thick or thin cuts, corresponding respectively to slices having relatively large thickness and zero-thickness slices of the breast being examined. After the image volume is reconstructed, 2D images corresponding to the image volume are reviewed by a radiologist in accordance with a predetermined reading protocol. However, the reading protocol may specify reviewing and analyzing a large number of images, which may be time consuming for a radiologist, and may consume computational and memory resources that could be used for other tasks. In many scenarios, a diagnosis may be made based on a smaller number of images.

### BRIEF DESCRIPTION

In one embodiment, a protocol generation system for a digital mammography system comprises a processor, and a memory storing instructions that when executed cause the processor to receive digital breast tomosynthesis (DBT) case images of a patient, the DBT case images including at least one projection set of images and/or image volume of a breast of the patient reconstructed from data acquired using the digital mammography system; generate a case score for the DBT case file by analyzing the case images; select a minimum dataset of the DBT case images to be read, based on the case score; and send the minimum dataset and/or a reading protocol specifying the minimum dataset to a radiology workstation (RWS) to be read with the dataset display sequence and/or store the minimum dataset in an archive.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1 is a schematic illustration of a digital mammography system according to an embodiment;
FIG. 2 is a diagram illustrating a workflow for selectively sending portions of a DBT study to a workstation of a radiologist for review, according to embodiments disclosed herein;
FIG. 3 is a flowchart illustrating a method for selectively sending portions of the DBT study to the workstation of the radiologist, according to embodiments disclosed herein;
FIG. 4 is a flowchart illustrating a method for selecting portions of a DBT study received from a digital mammography system to be reviewed by the radiologist, according to embodiments disclosed herein;
FIG. 5 is a flowchart illustrating a method for encoding a minimum dataset of a DBT study to be reviewed in a Digital Imaging and Communications in Medicine (DICOM) tag of the DBT study, according to embodiments disclosed herein;
FIG. 6 is a flowchart illustrating a method for generating a minimum dataset of a DBT study to be reviewed using a full-field digital mammography (FFDM) image, according to embodiments disclosed herein;
FIG. 7 is a flowchart illustrating a method for selectively retrieving portions of a DBT study from an archive, according to embodiments disclosed herein;
FIG. 8 is an exemplary imaging system infrastructure used to support the methods of FIGS. 3-7, according to embodiments disclosed herein;
FIG. 9 shows an exemplary representation of a full DBT image dataset, as prior art; and
FIG. 10 shows an exemplary representation of breast density images that are displayed based on reading protocols selectively generated by a protocol generation system, according to embodiments disclosed herein.

### DETAILED DESCRIPTION

The following description relates to various embodiments for digital mammography imaging procedures. Mammography is a medical imaging procedure for detecting one or more tumors of a breast. Based on mammography imaging, a breast biopsy procedure may be performed to obtain a biopsy sample of the concerned breast tissue for further analysis. During a breast biopsy procedure, the breast is compressed with a compression paddle, and is positioned in either a mediolateral or craniocaudal position, depending on whether the biopsy is performed in a horizontal approach, where the needle is inserted into the tissue along a mediolateral plane parallel to the detector, or a vertical approach, where the needle is inserted vertically along a craniocaudal plane. Location of a target tissue (e.g., lesion, microcalcification, etc.) is then identified based on a mammography imaging procedure, such as digital breast tomosynthesis (DBT). For example, during DBT, a scout image (where x-ray tube is in a midline position perpendicular to the detector) and a plurality of stereo images (where the x-ray tube moves in an arc at various angles within a set degree from the midline in both the positive and negative directions) are obtained. The target location within a region of interest (ROI) may be selected based on the acquired images. Upon selecting the target, a needle is inserted into the breast using a biopsy tool, and a portion of the target tissue is excised with the needle to obtain the biopsy sample.

Digital mammography imaging procedures may include acquiring two-dimensional (2D) or 3D digital images of the breast. For example, DBT is an imaging technique for generating cross-sectional images of a breast at high in-plane resolution. During imaging using a digital mammography system, the breast is compressed and an x-ray source may be rotated around the breast within a range of angles in positive and negative directions from a medial position. Low dose x-ray projection images of the breast at each angle may be obtained at a detector. The projection images are then reconstructed as slice images of breast volume along the z-direction.

Some medical procedures, such as breast biopsies, may be carried out with assistance from contrast-enhanced imaging performed with a digital mammography system. Contrast-enhanced imaging includes the administration of a contrast agent, such as iodine, to an imaging subject (e.g., patient). The contrast agent may travel to the patient's vasculature, which may assist in biopsy target (e.g., lesion) visualization. Following administration of the contrast agent, dual energy images may be obtained at various points of the biopsy procedure, such as immediately after contrast agent injection and prior to anesthesia delivery, after anesthesia delivery, after biopsy needle insertion, after firing of the biopsy device, after sample collection, and/or after biopsy clip insertion. A dual energy image may be generated from two images, where the two images include a first image acquired with low radiation energy (termed a low energy image, or LE) and a second image acquired with high radiation energy (termed a high energy image, or HE). A digital subtraction process may be used to generate the dual energy (DE) image from the LE image and the HE image, such that background features are removed from the DE image and the contrast-enhanced features (e.g., the lesion) are more accurately visualized.

When DBT is performed, one or more two-dimensional (2D) images may also be relied on to increase an accuracy of interpretation and for comparison with prior mammograms. The 2D image may be generated using full-field digital mammography (FFDM), or the 2D image may be generated using synthetic mammography (SM), where a 2D reconstruction of a tomosynthesis slice dataset is performed, to reduce image acquisition time and radiation exposure (in comparison with FFDM). The 3D image volume generated from the DBT and the 2D image may be read by a radiologist on a flat screen based on a reading protocol. The reading protocol, also referred to as a hanging protocol, may specify the display sequence of images to be read. As an example, a synthetic 2D (S2D) mammogram of four standard screening views might be displayed first. Then, following a user action, slabs are automatically displayed for the four standard views followed by planes. Such a reading protocol may be referred to as S2D + Planes + Slabs.

However, an S2D + Planes + Slabs reading protocol may entail reviewing and analyzing a large number of images (e.g., 244 images, in one example). Reading the large number of images may be time consuming for a radiologist, and may consume computational and memory resources that could be used for other tasks. A different reading protocol may be used, such as an S2D + Planes protocol, where slabs are not read, or an S2D + Slabs protocol, where planes are not read. However, following such protocols may still include viewing a lot of data. In some scenarios, an S2D-only reading protocol may be used without compromising reader performance, when S2D is sufficiently reliable to show diagnostically relevant information from an image volume in a single 2D image. However, nearly 100% of the diagnostic information of the image volume would have to be captured by an underlying S2D selection process, and setting an operating point of an S2D selector to ~100% sensitivity would lead to a high number of false positives, which may impair S2D image quality.

To address these issues, a protocol generation system is disclosed herein that takes a hybrid approach to protocol selection, where the protocol generation system performs an assessment of the 3D image volume, and selectively provides one or more reading protocols based on the assessment. An S2D-only protocol may be provided to the reader for cases that are easier to diagnose, or where there is high confidence in an automated analysis by an AI agent, and S2D + Slabs/Planes protocols may be provided for more difficult cases, where there is less confidence in automated analyses. Several embodiments are proposed, from hybrid reading protocols to smart hanging protocols. In this way, the protocol generation system provides an automated way of adjusting an amount of data to review, to speed up overall radiologist throughput. By adopting this hybrid approach, an amount of time spent by a radiologist reading DBT and synthetic 2D images may be reduced, without compromising reader performance. By reducing the amount of time spent reading the images, an amount of computational and memory resources of a computing system used to read the images may be reduced.

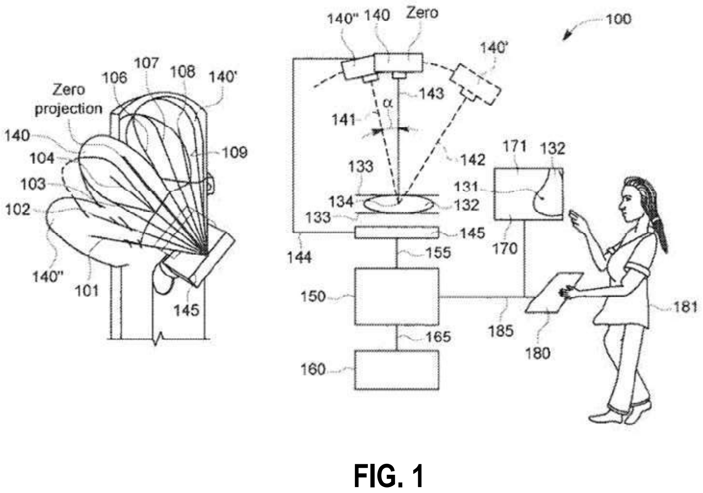

Referring now to the figures, FIG. 1 is a diagrammatic illustration of a digital mammography system 100 for obtaining an enhanced projection image of an object of interest, such as a breast, wherein digital mammography system 100 comprises a X-ray source 140 facing an X-ray detector 145. The X-ray source 140 and the X-ray detector 145 are connected by an arm 144. Between the X-ray detector 145 and the X-ray source 140 an object of interest 132 can be placed. In the system illustrated, the X-ray source 140 moves in an arc above the X-ray detector 145. The X-ray detector 145 and a plurality of positions of the X-ray source 140' and 140" following an arc (see dashed line) are shown with dashed/solid lines and in a perspective partial view. In the shown arrangement, the X-ray detector 145 is fixed at the shown position and only the X-ray source 140 moves. However, in other embodiments, the X-ray detector 145 may move as well, such that the X-ray detector 145 follows the movement of the X-ray source 140 and is substantially perpendicular to the orientations 141, 142 and 143. The angle α is a projection angle enclosed by the zero-orientation and any other orientation such as 141 and 142. In this way multiple different views of the breast tissue can be acquired via the at least one X-ray source 140. The projection of lowest α or the projection closest to the zero-orientation is named the central projection or zero projection by approximation.

Still referring to FIG. 1, on the left side is shown a partial perspective view of an imaging system according to an exemplary embodiment of the disclosure comprising an X-ray detector 145 and an X-ray source 140. The different positions of the X-ray source 140, 140' and 140" are broadly depicted to illustrate the movement of the X-ray source. There are nine different projection views 101, 102, 102, 103, 104, 106, 107, 108, 109, including the zero projection (105) indicated as straight lines, which all point to the center of the X-ray detector 145.

The patient (not shown) is positioned in front of the digital mammography system. To take for example, a mediolateral oblique (MLO) acquisition or view, a user or healthcare professional, such as a mammography technologist 181 will set the angle for the desired projection (30 degrees to 60 degrees, wherein 45 degrees represents the preferred zero projection shown in the perspective view of FIG. 1). During routine screening mammography, the angled MLO view is preferred over a lateral 90-degree projection because more of the breast tissue can be imaged.

The object of interest 132 shown in display unit 170 is a breast compressed by compression paddles 133, which ensure uniform compression and immobilization of the breast during the radiation exposure for optimal image quality. The breast 132 may include, for example, a calcification or lesion 131, which is located in the zero orientation 143, which is perpendicular to the detector 145 plane. The user may review calcifications, lesions or other clinically relevant structures for diagnosis. The display unit 170 may show a 2D mammography image, where mainly the middle portion of the breast 132 can be reviewed.

The X-ray detector 145 and the X-ray source 140 comprise an acquisition unit, which is connected via a data acquisition line 155 to a processing unit 150. The processing unit 150 comprises a memory unit 160, which may be connected to the processing unit 150 via an archive line 165.

The user or healthcare professional, such as the mammography technologist 181 may input control signals via a user interface 180. Such control signals are transferred from the user interface to the processing unit 150 via a signal line 185. The method and system according to the disclosure enables the user to obtain an enhanced 2D projection image that looks like a 2D mammogram. Based on this high-quality image, a radiologist is capable of identifying all the clinical signs relevant for breast screening. Especially if the user or healthcare professional is used to 2D mammograms, the user may easily analyze the displayed image. Further there is the possibility of displaying previously acquired 2D mammograms for comparison with an image acquired through a tomosynthesis imaging system according to the present disclosure. Besides, tomosynthesis images may be reviewed and archived. A CAD system or the user can provide 3D marks on images. A height map of clinically relevant structures or other objects obtained according to an embodiment of the disclosure can be combined with height information provided by 3D marks by a CAD system or indicated by a user through a 3D review system.

The memory unit 150 can be integral to or separate from the processing unit 150. The memory unit 160 allows storage of image data such as 2D enhanced projection images and tomosynthesis 3D images. In general, the memory unit 160 may comprise a computer-readable medium for example a hard disk or a CD-ROM, diskette, a ROM/RAM memory, DVD, a digital source such as a network or the Internet or any other suitable means. The processing unit 150 is configured to execute program instructions stored in the memory unit 160, which cause the computer to perform the methods of the disclosure. One technical effect of performing the method according to the embodiments of the invention is that the X-ray source may be less used, since the enhanced 2D projection images can replace a 2D mammogram, which usually is based on a separate X-ray exposure in order to acquire high-quality images.

After one or more DBT volumes and/or one or more mammograms have been generated by the digital mammography system 100, the volumes and/or mammograms may be sent to a radiologist to be read. The volumes and/or mammograms may provide a set of 2D images that will be read by the radiologist, in accordance with a reading protocol. The reading protocol may specify the set of 2D images to be sent to the radiologist, along with details specifying how or in what order the 2D images should be read. The reading protocol may be automatically selected by the digital mammography system 100, based on case criteria. The reading protocol may be a S2D + Planes + Slabs reading protocol, which specifies that the radiologist read a synthetic 2D image generated from the DBT volume, a number of images corresponding to various slices of the DBT volume, and a number of images corresponding to various slabs of the DBT volume (e.g., a plurality of adjacent slices) of a defined thickness. In some cases, the reading protocol may be an S2D + Planes reading protocol, or a S2D + Slabs protocol. The radiologist may read the 2D images specified by the reading protocol to make a diagnosis with respect to the patient.

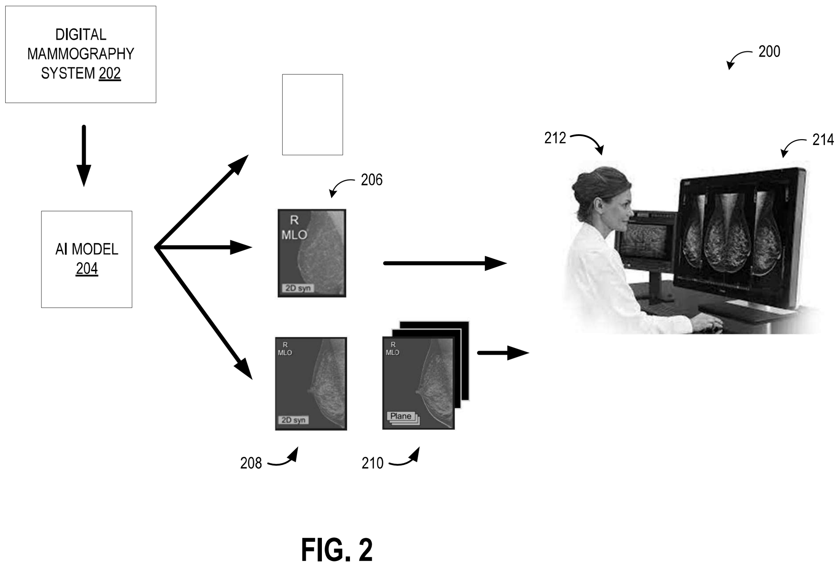

Because the total number of 2D images read by the radiologist may be large, and because reading the 2D images may therefore be time consuming, a protocol generation system may advantageously be used to select a reading protocol, a hybrid reading protocol, or a plurality of reading protocols based on a specific patient case, as described below in reference to FIGS. 2, 8, and 3.

Referring now to FIG. 2, an exemplary simplified workflow 200 is shown for generating a reading protocol and a set of DBT/mammography images of a breast of a patient to be sent to a radiologist for reading. The DBT/mammography images may be generated by a digital mammography system 202, such as the digital mammography system 100 of FIG. 1. A DBT volume reconstructed by the digital mammography system 202 may be inputted into an artificial intelligence (AI) model 204, which may analyze the volume to determine whether one or more tumors are present in the volume, and to further determine characteristics of the one or more tumors (e.g., extent, stage, whether the tumors are benign or malignant, type of tumors, etc.). The AI model may output a classification of the patient case. Based on the classification of the patient case, a reading protocol and a minimum dataset of images to be reviewed may be provided to a radiologist 212. For example, in response to a first classification indicating that there are no findings, no minimum dataset may be generated, and 2D images of the patient case may not be sent to a review workstation of the radiologist. In response to a second classification indicating that non-cancerous anomalies were detected, a first reading protocol may be sent for reading a first minimum dataset comprising an S2D image 206. In response to a second classification indicating that potentially cancerous anomalies were detected, a second reading protocol may be sent for reading a second minimum dataset comprising an S2D image 208 and a plurality of DBT images 210. In other embodiments, a different reading protocol and a different minimum dataset may be generated. The images sent to the radiologist 212 may be reviewed by the radiologist 212 on a display screen 214 of a computing device of the radiologist, such as a remote workstation (RWS).

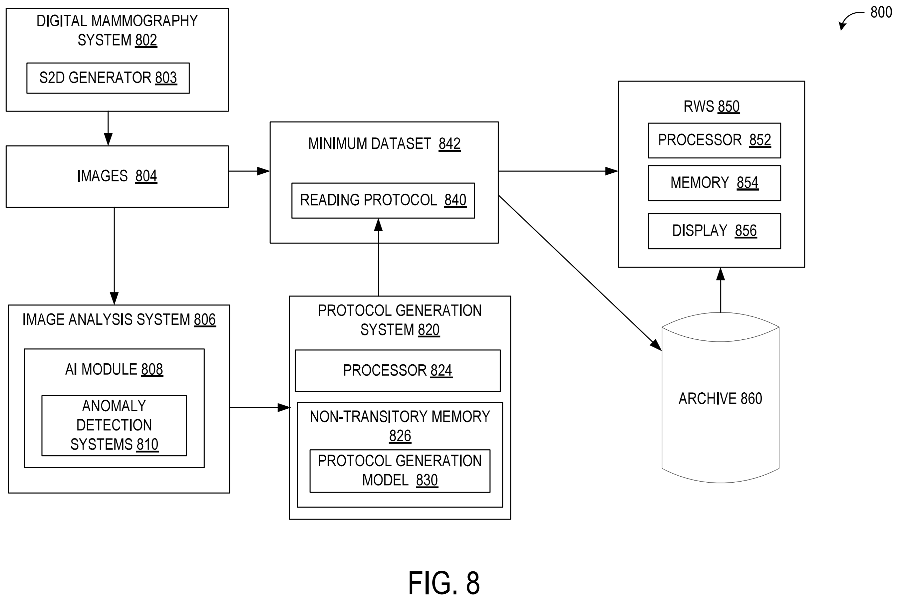

FIG. 8 shows an infrastructure diagram 800 of an exemplary imaging system infrastructure used to support workflow 200, as well as workflows of other embodiments described herein. Infrastructure diagram 800 includes a digital mammography system 802, which may be a non-limiting version of digital mammography system 100 of FIG. 1. Digital mammography system 802 may generate one or more images 804 of a breast of a patient, including DBT volumes and/or 2D FFDM images. Images 804 may also include an S2D image, which may be generated from a DBT volume by an S2D generator 803. S2D generator 803 may be or include an anomaly detection system used to generate a set of slabs, a set of planes, and/or an S2D image from the one or more images 804.

Images 804 may be sent to an image analysis system 806, which may be integrated into digital mammography system 802 or may be installed at a different computing device. Image analysis system 806 includes an AI module 808, which may include a plurality of AI-based anomaly detection systems 810. The plurality of AI-based anomaly detection systems 810 may include, for example, a risk assessment system; a negative triage system; a breast density assessment system; an anomaly detection system used to generate a set of slabs, a set of planes, and/or an S2D image from DBT case images; and/or a different anomaly detection system.

One or more anomaly detection systems 810 may be used to process the images 804, to detect lesions, tumors, calcifications, and/or other anomalies. That is, the one or more anomaly detection systems 810 may take image data of images 804 as input, and may output an encoding of one or more findings, where the findings may include detected lesions, tumors, etc. The findings may include, for example, a location of an anomaly in the image data; a classification of the anomaly; an assessment of a seriousness of the anomaly; and/or a confidence score indicating a degree of confidence of the anomaly detection system 810 in assessing the seriousness of the anomaly.

The findings detected by the one or more anomaly detection models 810 may be sent to a protocol generation system 820. In the embodiment depicted in FIG. 8, protocol generation system 820 is installed on a separate computing device communicatively coupled to image analysis system 806. In other embodiments, protocol generation system 820 may be integrated into digital mammography system 802, or image analysis system 806.

Protocol generation system 820 includes a processor 824 configured to execute machine readable instructions stored in non-transitory memory 826. Processor 824 may be single core or multi-core, and the programs executed thereon may be configured for parallel or distributed processing. In some embodiments, processor 824 may optionally include individual components that are distributed throughout two or more devices, which may be remotely located and/or configured for coordinated processing. In some embodiments, one or more aspects of processor 824 may be virtualized and executed by remotely-accessible networked computing devices configured in a cloud computing configuration.

Non-transitory memory 826 includes a protocol generation model 830, which may be a classification model used to classify an output of image analysis system 806. Protocol generation model 830 may take the encoding of one or more findings of images 804, and output a classification of the one or more findings. For example, if there are no findings, protocol generation model 830 may output a first classification (e.g., a 0). If findings are detected that can be diagnosed with a high degree of confidence (e.g., a benign tumor), protocol generation model 830 may output a second classification (e.g., a 1). If findings are detected, and the findings are more serious and/or not as easily diagnosed (e.g., a malignant tumor), protocol generation model 830 may output a third classification (e.g., a 2). In other embodiments, a greater or lesser number of classifications, or different types of classifications may be outputted by protocol generation model 830.

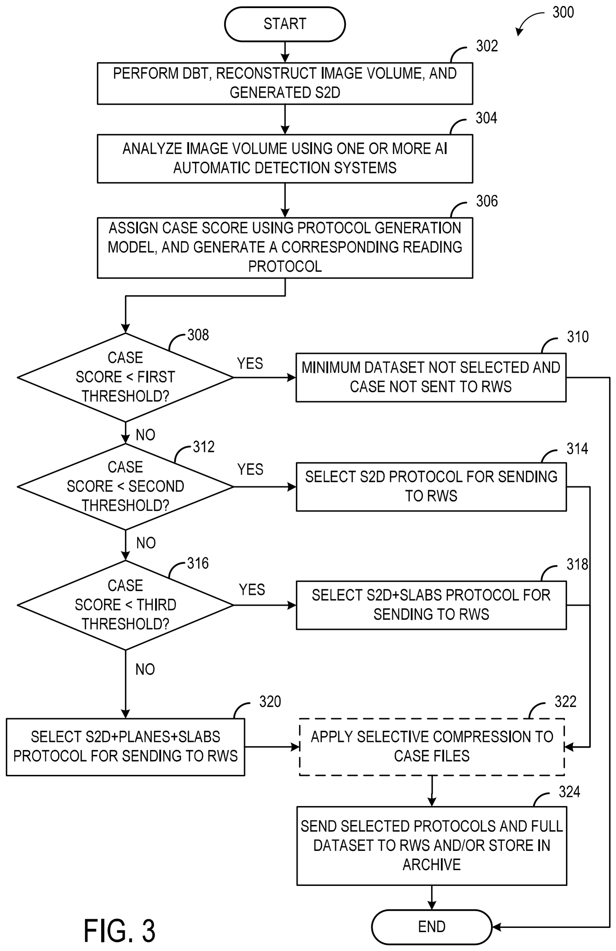

Based on the output of protocol generation model 830, protocol generation system 820 may generate a reading protocol 840 for reading images 804. However, unlike typical reading protocols for reading images 804, reading protocol 840 may specify a minimum dataset 842 to be read by a radiologist, where minimum dataset 842 may include a smaller number of images than images 804. Minimum dataset 842 and reading protocol 840 may be sent to an RWS 850 via a network, such as a wireless network 870, to be displayed to a radiologist. When minimum dataset 842 and reading protocol 840 are received at RWS 850, minimum dataset 842 and reading protocol 840 may be stored in a memory 854 of RWS 850, and may be displayed on a display 856 of RWS 850 based on instructions included in reading protocol 840 and executed on a processor 852 of RWS 850. As described in greater detail below in FIGS 3-8, reading protocol 840 may advantageously reduce a number of images to be sent to the radiologist for reading, to reduce a consumption of bandwidth of minimum dataset 842 and reading protocol 840 on network 870.

Additionally or alternatively, in some embodiments, minimum dataset 842 and reading protocol 840 may be sent to an archive 860 for storage, and retrieved by RWS 850 from archive 860. That is, minimum dataset 842 and reading protocol 840 may not be sent to RWS 850, and minimum dataset 842 and reading protocol 840 may be retrieved from archive 860 by RWS 850. By retrieving minimum dataset 842 and reading protocol 840 from archive 860, a prefetching strategy may be employed to efficiently manage a bandwidth consumption of minimum dataset 842 and reading protocol 840 over a network, as described below in reference to FIG. 8.

Referring now to FIG. 3, a flowchart of an exemplary method 300 is shown for generating a reading protocol to send to a radiologist, in accordance with the workflow 200 described above. Method 300 may be executed using computer readable instructions stored in the non-transitory memory of computing device of a digital mammography system (e.g., digital mammography system 100 of FIG. 1) or a controller communicatively coupled to the digital mammography system (e.g., controller 44 of FIG. 1). In some embodiments, the protocol generation system may be installed and operated on another computing device without departing from the scope of this disclosure (e.g., an edge device, a picture archiving and communication system (PACS)).

Method 300 begins at 302, where method 300 includes performing a DBT scan on a patient, using the digital mammography system. An image volume may be reconstructed from projection data acquired during the DBT scan. For the purposes of this disclosure, patient data, image data, and results of any analyses performed on the image data may be referred to collectively as the patient case. Additionally, an S2D image may be generated from the image volume, in accordance with various techniques known in the art. The S2D image generation process may rely on an automatic analysis of a DBT volume generated from the scan seeking lesions. Once findings' positions are detected, a rendering process generates a 2D image out of the DBT acquisition with a specific emphasis on findings.

At 304, method 300 includes analyzing the image volume using one or more AI models, such as an anomaly detection model of anomaly detection systems 810 of FIG. 8. The AI models may include, for example, statistical models, rules-based models (e.g., decision trees, Bayesian networks, etc.), and/or machine learning (ML) or deep learning (DL) models. For example, the AI models may include a convolutional neural network (CNN) trained to detect lesions or anomalies in an image volume of a breast. The one or more AI models may take the image volume as input, and may output an assessment of the breast. The assessment may include a determination of whether a lesion was detected, and an estimation of the severity and/or extent of the lesion, type of lesion (cluster of calcification or mass), for example.

At 306, method 300 includes assigning a case score to the patient case based on the aggregation of the assessment for each acquisition of the patients breasts, and generating a corresponding reading protocol and minimum dataset of images to review in accordance with the reading protocol. The case score may classify the assessment into one of a number of categories or classifications. The case score may be integer value, such as a one, a two, etc. The case score may be assigned by a protocol generation system, such as protocol generation system 820 of FIG. 8. Specifically, the case score may be generated by a protocol generation model (e.g., protocol generation model 830) of the protocol generation system. The protocol generation model may take as input the assessments of each acquisition of the patient's breasts, and output the case score. In some embodiments, the case score may instead be received as an input from an outside source by the proteocal generation system 820. Based on the case score, a suitable reading protocol and minimum dataset may be selected.

At 308, method 300 includes determining whether the case score is less than a first threshold. For example, the case score may be a zero, and the first threshold may be a one. If the case score is less than the first threshold, it may be deduced that no anomalies were detected in the breast by the one or more AI models with a high degree of confidence, whereby method 300 proceeds to 310. At 310, method 300 includes not selecting a minimum dataset, not sending the patient case to an RWS of a radiologist (e.g., radiologist 312), and method 300 ends.

Alternatively, if the case score is not less than the first threshold, method 300 proceeds to 312. At 312, method 300 includes determining whether the case score is less than a second threshold. For example, the case score may be a one, and the first threshold may be a two. If the case score is less than the second threshold, it may be deduced that the patient case includes findings detected and diagnosed by the one or more AI models with a high degree of confidence, whereby method 300 proceeds to 314. At 314, method 300 includes selecting a minimum dataset including the S2D image and an S2D protocol (e.g., an S2D-only protocol) to send to the RWS of the radiologist, and method 300 proceeds to 322. The S2D-only protocol may indicate that the radiologist confirm the findings of the one or more AI models based on the S2D image, but may not specify that the radiologist review the DBT images. However, the DBT images, the DBT volume, and/or other data or files of the patient case may be additionally sent to the radiologist for reference purposes.

Alternatively, if at 312 the case score is not less than the second threshold, it may be inferred that the patient case includes findings detected and diagnosed by the one or more AI models with a lower degree of confidence and/or a higher degree of severity, where a detailed examination and confirmation of the diagnosis of the one or more AI models by a human expert is desired. For example, one or more malignant tumors may be detected in the breast by the one or more AI models.

If at 312 the case score is not less than the second threshold method, method 300 proceeds to 316. At 316, method 300 includes determining whether the case score is less than a third threshold. If the case score is less than the third threshold, method 300 proceeds to 318. At 318, method 300 includes selecting a minimum dataset including the S2D image and the slabs, and an S2D+Slabs protocol to send the RWS of the radiologist, and method 300 proceeds to 322. The S2D+Slabs protocol may specify that the radiologist confirm the findings of the one or more AI models using the S2D and the slabs, but may not specify that the radiologist review the full set of DBT images. However, the DBT images, the DBT volume, and/or other data or files of the patient case may be additionally sent to the radiologist for reference purposes.

Alternatively, if at 316 the case score is not less than the third threshold, it may be inferred that the patient case includes findings detected and diagnosed by the one or more AI models with an even lower degree of confidence and/or an even higher degree of severity, where a more detailed examination and confirmation of the diagnosis of the one or more AI models by a human expert is desired. If at 316 the case score is not less than the third threshold, method 300 proceeds to 320. At 320, method 300 includes selecting an S2D+planes+slabs protocol to be sent to the radiologist for review, where the minimum dataset is the full dataset of images of the patient case. The protocol may specify that the radiologist confirm the findings, based on the full dataset.

At 322, method 300 optionally includes applying a selective compression algorithm to the selected case files, meaning, the case files selected at 314 for the S2D-only protocol, the case files selected at 318 for the S2D+slabs protocol, or the case files selected at 320 for the S2D+planes+slabs protocol. The selective compression algorithm may advantageously compress different case files for transmission to the RWS and/or to an archive (e.g., archive 860) of the digital mammography system in different ways, to increase an efficiency of the transmission and/or to reduce an amount of time taken to send the case files to the RWS or archive and/or reduce an amount of storage space of the RWS or archive. In particular, the selected case files may include the minimum dataset to be read (e.g., specified by a relevant reading protocol), and additional case files that may be used for reference purposes. For example, for patient cases with case scores greater than the first threshold and less than the second threshold, the minimum dataset may include the S2D images, and the additional case files may include the full dataset of DBT images. The radiologist may receive and read the S2D images in accordance with the S2D-only protocol, and may optionally consult the full dataset of DBT images during an examination of the S2D images.

To increase the efficiency of the transmission and/or to reduce the amount of time taken to send the case files to the RWS and/or to reduce an amount of storage space of the RWS, the minimum dataset may be compressed using a first compression strategy, and the additional case files or remaining files of the full dataset may be compressed using a second compression strategy. For example, the first compression strategy may be a lossless compression strategy, and the second compression strategy may be a lossy compression strategy. By using the lossless compression for the files specified in the reading protocol, and using the lossy compression for the additional files not specified in the reading protocol, a size of the total amount of data transmitted to the RWS may be reduced, in comparison with an alternative situation where all of the case files are transmitted with lossless compression. By reducing the total amount of data transmitted to the RWS, a consumption of bandwidth during the transmission may be advantageously reduced, which may allow for more rapid and efficient flow of information throughout networked computing devices associated with the digital mammography system and a more rapid communication between the devices. Additionally, an amount of memory used to store the transmitted case files on the RWS may be reduced, which may increase an amount of memory available for storing other data.

At 324, method 300 includes sending the selected protocols and full dataset to the RWS and/or storing the selected protocols and full dataset in the archive, and method 300 ends.

Thus, based on an output of the one or more AI models, a different reading protocol and a different set of images referenced by the protocol may be sent to the radiologist. When there are no findings and a diagnosis of the patient may be straightforward, the radiologist may not be asked to read the images. When there are findings but the diagnosis may be uncomplicated and malignancy is not suspected, a first protocol may be sent to the radiologist that specifies reading the S2D image and not other images, where the diagnosis of the one or more AI models may be confirmed by the radiologist using the S2D image without relying on other images of the breast extracted from the DBT volume. When the findings indicate a malignant tumor or a more complicated diagnosis, a second or third protocol may be sent to the radiologist, where the second or third protocol may specify reading additional images or a full set of images to determine whether the findings are confirmed. In this way, the AI models are advantageously used to reduce a number of images reviewed by the radiologist, in cases where a diagnosis may be made without reviewing a full set of DBT images. By reducing the number of images reviewed by the radiologist in these cases, a time spent by the radiologist and an amount of system resources consumed during the review may be reduced.

In other words, the one or more AI models are used to define a minimum dataset that can be relied on by a radiologist when making a diagnosis. The minimum dataset may comprise a single S2D image, a full set of DBT images, or a partial set of DBT images, where the DBT images include both slab images and/or plane images. Further, in various embodiments, remaining image sets that are not part of the minimum dataset may additionally be sent to the radiologist, and an option may be provided to display the remaining image sets that are not part of the minimum dataset on the display device. To conserve resources and bandwidth, the minimum dataset may be stored using lossless compression, and the remaining image sets may be stored using lossy compression.

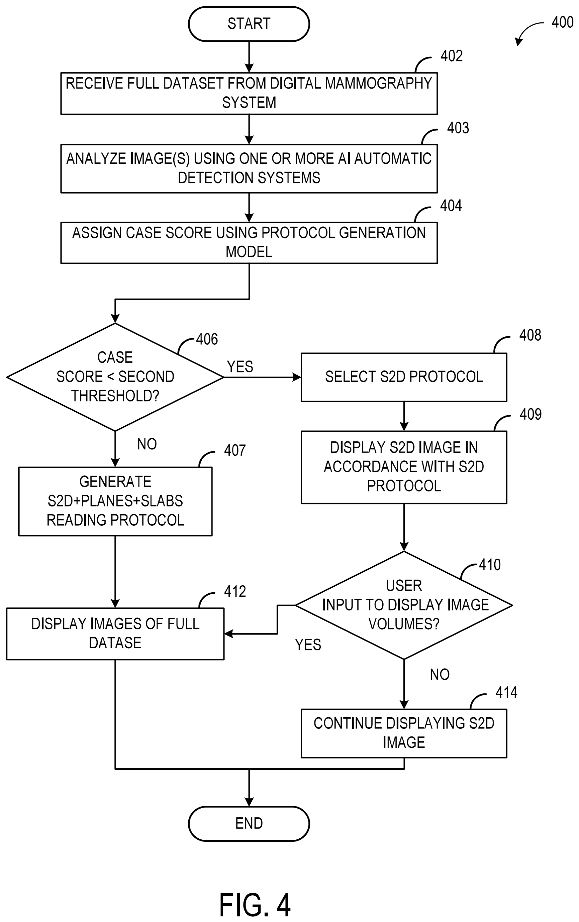

Another embodiment is described in FIG. 4, where the protocol generation system is installed and operated at the RWS of the radiologist. FIG. 4 shows a method 400 for generating a reading protocol based on a full dataset of a patient case received from a digital mammography system, such as digital mammography system 100 of FIG. 1. In contrast with method 300 of FIG. 3, method 400 may be executed by a processor of the RWS based on instructions stored in a memory of the RWS, such as processor 852 and memory 854, respectively, of FIG. 8.

Method 400 begins at 402, where method 400 includes receiving the full dataset of the patient case from the digital mammography system. The full dataset may include patient data, a set of 2D images and/or 3D image volumes reconstructed by the digital mammography system, and one or more outputs of one or more AI (anomaly detection) models.

At 403, method 300 includes analyzing one or more images of the full dataset using one or more AI models, such as an anomaly detection model of anomaly detection systems 810 of FIG. 8. As described above in reference to method 300, the AI models may include, for example, statistical models, rules-based models (e.g., decision trees, Bayesian networks, etc.), and/or machine learning (ML) or deep learning (DL) models. For example, the AI models may include a convolutional neural network (CNN) trained to detect lesions or anomalies in an image volume of a breast. The one or more AI models may take the image volume as input, and may output an assessment of the breast. The assessment may include a determination of whether a tumor was detected, and an estimation of the severity and/or extent of the tumor, for example.

At 404, method 400 includes assigning a case score to the patient case, using the protocol generation model, as described above in reference to method 300. At 406, if the case score is not less than the second threshold, it may be inferred that the patient case includes findings detected and diagnosed by the one or more AI models with a lower degree of confidence and/or a higher degree of severity, where a detailed examination and confirmation of the diagnosis of the one or more AI models by a human expert is desired. As such, method 400 proceeds to 407, where method 400 includes selecting an S2D+planes+slabs reading protocol for reading the images of the full dataset, and at 412, method 400 includes displaying the full dataset of images in accordance with the S2D+planes+slabs reading protocol. The images of the full dataset may be displayed via a hanging protocol established within the reading protocol specifying a display sequence of the images of the minimum dataset. In other embodiments, a third threshold may be similarly used to send an S2D+slabs reading protocol and minimum dataset as described in reference to method 300, which for simplicity is not shown here.

Alternatively, if at 406 the case score is less than the second threshold, method 400 proceeds to 408. At 408, method 400 includes selecting a S2D-only reading protocol for displaying the S2D images. As with method 300, if the case score is less than the second threshold, it may be deduced that the patient case includes benign findings detected and diagnosed by the one or more AI models with a high degree of confidence, where the S2D image may be sufficient for confirming the diagnosis of the one or more AI models. At 409, method 400 includes displaying the S2D image in accordance with the S2D reading protocol.

At 410, if an input is received from a user of the RWS (e.g., radiologist 112) requesting to view other images of the full dataset, method 400 proceeds to 412, where additional images of the full dataset may be displayed. In such cases, no hanging protocol may be established, and the user may select desired individual images of the additional images to display. If at 410 no user input is received for viewing the other images of the full dataset, method 400 proceeds to 414, where method 400 includes continuing to display the S2D images in accordance with the S2D image protocol. Method 400 ends.

While the embodiment of method 400 consumes more bandwidth during the transmission of the full dataset from the digital mammography system to the RWS, an advantage of method 400 over method 300 is that processing of the output of the AI models used for anomaly detection to generate the reading protocols is performed using a processor of the RWS, rather than a processor of the digital mammography system. Because the digital mammography system may generate a plurality of images that are sent to a plurality of RWSs, by transferring the processing to edge devices, an overall computational efficiency of the digital mammography system may be increased, and processing power of the digital mammography system may be freed for other tasks. Another benefit of method 400 over method 300 is that the radiologist can manually request the display of data that is not part of the minimum dataset. This might be helpful in the event that the AI system fails at properly identifying the minimum dataset to be read.

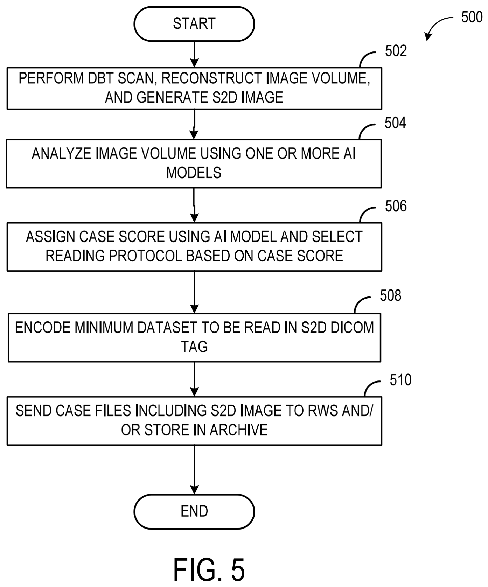

Another embodiment is shown in FIG. 5, which shows an alternative method 500 for generating a reading protocol and selectively sending data files to a receiving RWS. Method 500 may be performed by a processor of the digital mammography system, or a computing device coupled to the digital mammography system. Method 500 starts at 502, where a DBT scan is performed, a volume is reconstructed from data of the DBT scan, and an S2D image is generated. At 504, method 500 includes analyzing the volume using one or more AI models used to generate the S2D images, and at 506, a case score is assigned based on the output of the one or more AI models used to generate the S2D images for each DBT acquisition of the patient case, and selecting a reading protocol based on the case score. However, in contrast with method 300, at 508, the minimum dataset to be read and the associated reading protocol is encoded into a Digital Imaging and Communications in Medicine (DICOM) tag of the S2D image. In some examples, the output of the one or more AI models may also be encoded into the DICOM tag.

At 510, the case files including the S2D images are sent to the receiving RWS or stored in an archive of the digital mammography system. When the case files are received at the receiving RWS, or any other RWS, the reading protocol, minimum dataset, and/or output of the one or more AI models may then be extracted and decoded from the DICOM tag, and the relevant images may be displayed in accordance with the extracted reading protocol.

By encoding the reading protocol in the DICOM tag, any requesting RWS may receive the full dataset, and extract the reading protocol to determine the specific images of the full dataset to display. That is, the generation of the reading protocol is performed once, and any subsequent requests for case files will include a respective reading protocol for viewing the case files. In this way, processing resources of the digital mammography system may be reduced, making the digital mammography system more efficient and increasing a number of computational tasks that can be performed on the digital mammography system.

In some medical scenarios, a combo acquisition may be performed, where 2D FFDM images are generated by the digital mammography system in addition to DBT images. Some anomalies, such as calcifications, may be more easily seen in FFDM images than in DBT images. In another embodiment, FFDM image data may be an additional input into the protocol generation model used to determine an appropriate reading protocol for reading images of either or both of the DBT images and the FFDM images.

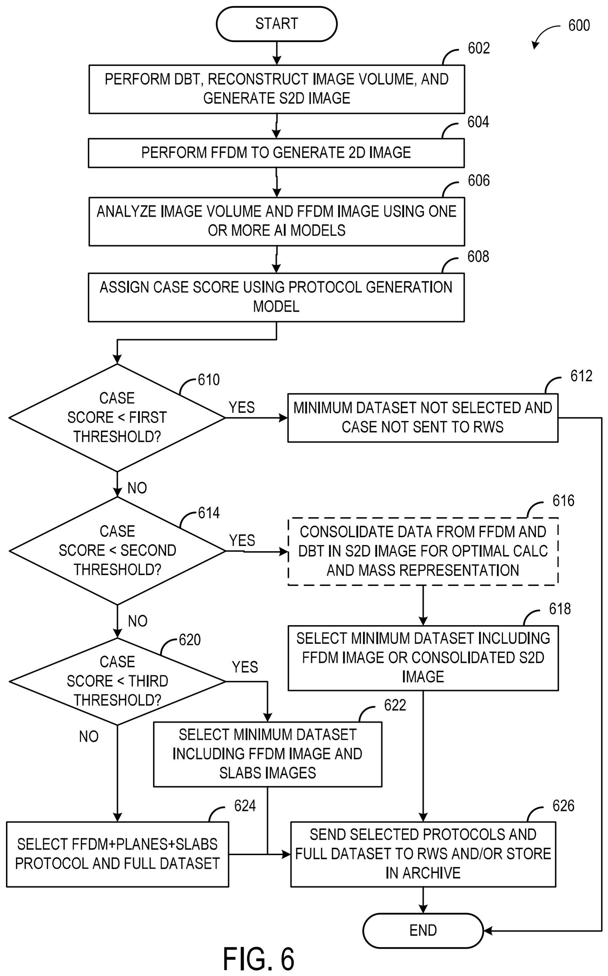

FIG. 6 shows a method 600 for generating a reading protocol and sending images included in the reading protocol to an RWS for viewing, where the images include both DBT images and FFDM images. Method 600 may be performed by a processor of the digital mammography system, based on instructions stored in a memory of the digital mammography system and/or a protocol generation system of the digital mammography system.

Method 600 begins at 602, where method 600 includes performing a DBT examination of a breast of a patient, reconstructing an image volume from data acquired during the DBT examination, and generating an S2D image from the image volume and/or DBT projections. At 604, method 600 includes performing an FFDM examination of the patient, to generate a 2D FFDM image. At 606, method 600 includes analyzing either or both of the DBT volume and the FFDM image using one or more AI models, as described above. The one or more AI models may take DBT data as input, FFDM data as input, or both DBT and FFDM data as input. In various embodiments, an AI model (e.g., of anomaly detection systems 810) may be used to detect and identify the calcifications in the FFDM images and/or DBT volumes. At 608, a case score may be assigned to the patient case using an output of the AI model. In particular, in addition to the case scored described above in reference to method 300, an additional case score may be created for a scenario in which calcifications are detected in the FFDM image.

At 610, method 600 includes determining whether the case score is less than a first threshold, as described above in reference to method 300. If the case score is less than the first threshold, at 612, a minimum dataset may not be selected, and the patient case may not be sent to the RWS for viewing. If the case score is not less than the first threshold, then method 600 proceeds to 614, where method 600 includes determining whether the case score is less than a second threshold. If the case score is less than the second threshold, then method 600 proceeds to 616.

At 616, method 600 optionally includes consolidating data from the FFDM image and the DBT volume in the S2D image, for optimal calcification and mass representation. If the data from the FFDM image and the DBT image are consolidated, at 618, method 600 includes selecting the consolidated S2D image as the minimum dataset and the S2D reading protocol. Alternatively, if the data from the FFDM image and the DBT image are not consolidated at 616, at 618, method 600 includes selecting the FFDM image as the minimum dataset and selecting an FFDM reading protocol.

If at 614 the case score is not less than the second threshold, then method 600 proceeds to 620. At 620, method 600 includes determining whether the case score is less than a third threshold. If the case score is less than the third threshold, the FFDM image may not be sufficient on its own, and method 600 proceeds to 622. At 622, method 600 includes selecting a minimum dataset including the FFDM image and the slabs, with an FFDM+slabs reading protocol.

If at 620 the case score is not less than the third threshold, then it may be inferred that calcifications were detected in the FFDM images, whereby method 600 proceeds to 624. At 624, method 600 includes selecting a minimum dataset including the FFDM image, the planes, and the slabs, and an FFDM+S2D+planes+slabs protocol for reading the full dataset of images of the patient along with the FFMD image. Preferably, the FFDM image may be prioritized for display over other images of the full dataset.

At 626, the method includes sending the selected protocol and the full dataset to the RWS for viewing and/or storing the selected protocol and the full dataset in an archive of the digital mammography system, and method 600 ends.

In other words, in some cases the FFDM images may include anatomical features (e.g., calcifications) that are not taken into account by some AI models used to detect anomalies in the DBT volume. That is, some AI models may be directed at detecting cancer, but not calcifications. By including FFDM image data as input into the protocol generation model, a case score may be generated that reflects findings of the FFDM images in addition to findings of the DBT volume. If the calcifications are detected, it may be advantageous for the radiologist to first view the calcifications in the FFDM images, and then proceed to review the S2D images and other images of the full dataset. Additionally, the FFDM image data may be consolidated into the S2D image, in which case the S2D-only protocol may be sufficient for reviewing the patient case, in some examples.

A further advantage of the systems and methods disclosed herein is that the reading protocols selectively generated by the protocol generation system may be used to expedite and increase an efficiency of pre-fetching image data from an archive, such as archive 860 of FIG. 8. As described above, in some embodiments, DBT and/or FFDM image data may not be sent to an RWS for reviewing by a radiologist; rather, the DBT and/or FFDM image data may be transmitted to an archive (e.g., a database, or dedicated space in memory) for storage. A reviewing radiologist may then request the image data from the archive, and the image data may be transmitted from the archive to an RWS of the reviewing radiologist. Transmitting the image data to the archive rather than the RWS may reduce an overall consumption of bandwidth during transmitting the image data from the digital mammography system (e.g., a scanner) to the RWS, and/or facilitate more efficient transmission of the image data. For example, the image data may be transferred from the digital mammography system to the archive via a first network, and the image data may be transferred from the archive to the RWS via a second network. The first network may be configured to maximize an efficiency of data transfers between the digital mammography system and the archive, and the second network may be configured to maximize an efficiency of data transfers from the archive to a plurality of networked RWSs. The archive may also be physically located in close proximity to the digital mammography system.

To increase an efficiency of data transfers from the archive to the plurality of RWSs, a pre-fetching strategy may be used, where image data associated with a patient case may be retrieved from the archive and stored on an RWS at a first time, based on a scheduled review of the patient case by a radiologist using the RWS. The image data may then be reviewed by the radiologist on the RWS at a second (e.g., scheduled) time after the first time. Additionally or alternatively, a first portion of the image data (e.g., the S2D image) may be retrieved at a first time, and additional image data (e.g., the full dataset) may be retrieved at the second time. By retrieving or pre-fetching the image data in advance, data transfers may be more efficiently managed across the plurality of RWSs. The data transfers may be scheduled for times when an availability of bandwidth is high; the data transfers may be performed at slower rates without impacting workflow; and so on.

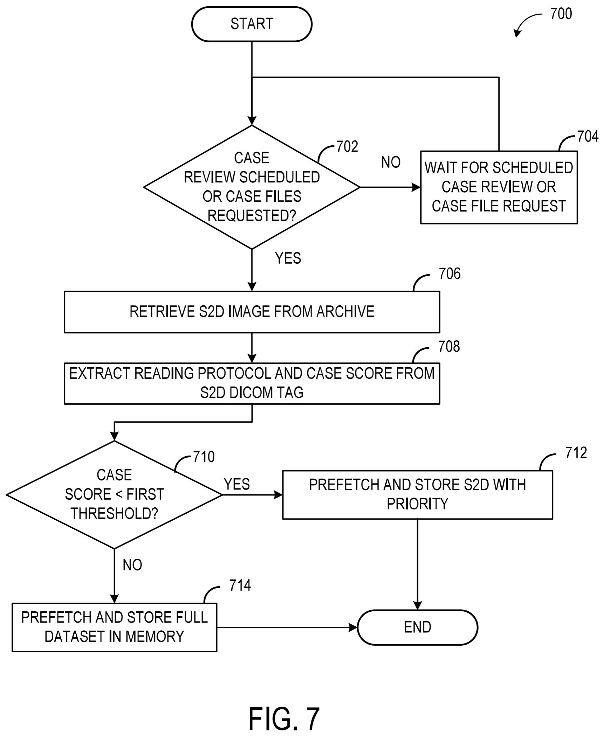

FIG. 7 shows a method 700 for pre-fetching DBT and/or FFDM images in a manner that advantageously uses the case scores generated by the protocol generation system to increase an efficiency of data transfers between the archive and a receiving RWS, according to an embodiment. In some embodiments, the case score may instead be received from an outside source. Method 700 may be performed by a processor of the RWS, based on instructions stored in a memory of the RWS (e.g., processor 852 and memory 854, respectively). In the embodiment described herein, method 500 of FIG. 5 may be performed as a precondition for executing method 700, where a reading protocol or minimum dataset to be read is determined by the protocol generation system at the digital mammography system, or at a server, prior to DBT and/or FFDM images being stored at an archive, and the reading protocol or minimum dataset is encoded in a DICOM tag of an S2D image for decoding at an RWS.

Method 700 begins at 702, where method 700 includes determining whether a patient case (including DBT and/or FFDM images) is scheduled for a review by a radiologist using the RWS, or whether case files regarding a patient of a digital mammography system have been requested. In some examples, the radiologist may schedule the review and/or make or schedule a request for case files of the patient case via a workflow management application installed on the RWS. In other examples, the patient case may be scheduled for review by the radiologist by a healthcare facility or management team, for example, via case management software running on a server of the healthcare facility.

If at 702 it is determined that a case review is not scheduled for review on the RWS, or that case files are not requested for a case review on the RWS, method 700 proceeds to 704, where method 700 includes waiting for a scheduled case review or case file request. Alternatively, if at 702 it is determined that a case review is scheduled for review on the RWS, or that case files are requested for a case review on the RWS, method 700 proceeds to 706.

At 706, method 700 includes retrieving the S2D images from the archive, and at 708, method 700 includes extracting a reading protocol and case score for the patient case from the DICOM tag of the S2D image files. In some embodiments, the case score may instead be received as an input from an outside source. As described earlier, the reading protocol may specify a set of image files to be reviewed as a priority by the radiologist. For example, the reading protocol may be an S2D reading protocol that specifies that the S2D image be read, where the S2D image may include sufficient data to confirm a previous diagnosis of the patient made by an AI algorithm. The reading protocol may specify that an FFDM image be read prior to reading other images, for example, if the FFDM image shows information (e.g., such as calcifications) relevant to the previous diagnosis that may not have been captured, or that may have been captured less clearly or accurately in the DBT volume. The reading protocol may be an S2D+planes+slabs protocol that specifies that a full dataset of DBT images be read, and may additionally specify an order in which individual DBT images should be read.

The case score generated by the protocol generation system or received from the outside source may indicate a type or classification of reading protocol included in the DICOM tag. For example, a first case score may indicate an S2D protocol; a second case score may indicate an S2D+planes+slabs protocol; a third case score may indicate that an FFDM image should be read; and so on.

At 710, method 700 includes determining whether the extracted case score is less than a first threshold. If the extracted case score is less than the first threshold, then it may be deduced that the reading protocol is an S2D-only protocol, where the S2D image may be read by the radiologist and other images of the full dataset may not be read by the radiologist. Method 700 proceeds to 712, where the S2D protocol and the S2D image may be uploaded and stored in the memory of the RWS with a high priority for later review by the radiologist, with the planes and/or slabs being uploaded with a lower priority. Alternatively, if at 710 it is determined that the case score is not less than the first threshold, it may be inferred that the full dataset may be read by the radiologist, whereby method 700 proceeds to 714. At 714, method 700 includes retrieving components of the full dataset of DBT images (and/or a DBT volume) from the archive with equal priority, and storing the reading protocol and full dataset in the memory of the RWS for later review by the radiologist.

It should be appreciated that method 700 represents a simplified procedure used to determine, based on the extracted reading protocol, whether to prioritize the S2D/FFDM images over the other DBT data. In other embodiments, method 700 may include additional steps, where based on different case scores, various types or subsets of images may be retrieved with different levels of priority. By retrieving images included in the minimum dataset with a higher priority, and retrieving images not included in the minimum dataset with a lower priority, an availability of bandwidth for transmitting different types of data may be managed more efficiently, increasing an overall efficiency of the digital mammography system in general.

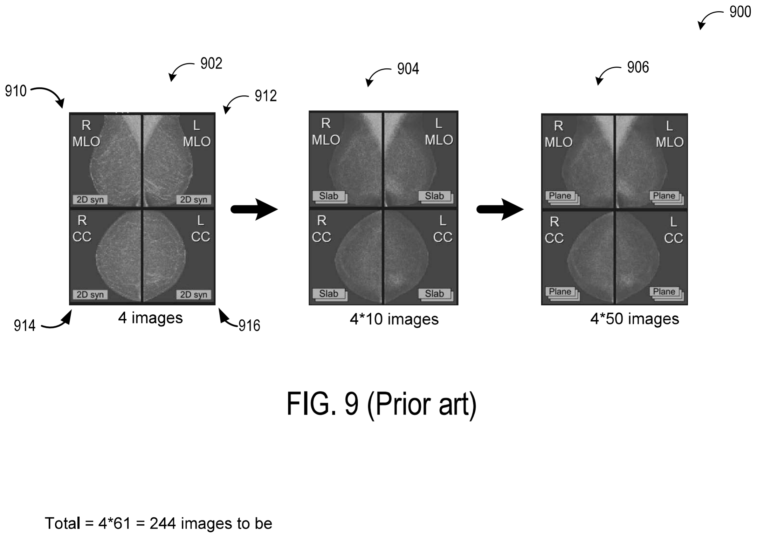

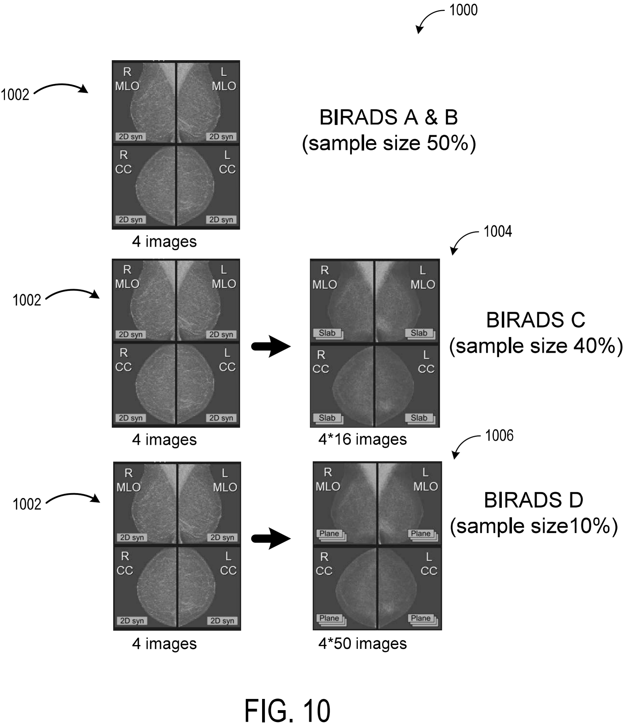

An exemplary quantification of a reduction in an amount of image data transmitted from a digital mammography system to an RWS as a result of applying the systems and methods described above is shown in FIGS. 9 and 10. Referring now to FIG. 9, an exemplary representation of a full image dataset 900 of a patient case is shown, representing a total amount of images that may typically be transferred to an RWS to be displayed (as prior art). The total amount of images includes an S2D image 902, which is a composite image comprising four separate images: a right mediolateral oblique image (RMLO) 910; a left mediolateral oblique image (LMLO) 912; a right craniocaudal image (RCC) 914; and a left craniocaudal image (LCC) 916. In addition to the S2D image 902, 50 RMLO, LMLO, RCC, and LCC slice images 906 may be transferred, based on a slice thickness of 1 mm, and an average breast thickness of 5 cm. In addition to the slice images 906, 16 slab images 904 may be transferred, where each slab image is generated from six consecutive slices, each slab image overlapping a previous slab image by 3 mm. As a result, a total number of images that would be transferred in a full dataset (e.g., under a S2D+planes+slabs reading protocol) would be 268 images (e.g., 4 images for the S2D image 902; 64 images for the slab images 904; and 200 images for the slice images 906).

In contrast, FIG. 10 shows an exemplary representation 1000 of a number of images that may be transferred to an RWS to be displayed when the reading protocols are selectively generated by the protocol generation system described herein. Exemplary representation 1000 is based on an assignment of case scores and reading protocols based on breast density, where images of breasts that are fattier (e.g., less dense) may be easier to diagnose as healthy than breasts that are more dense. That is, a first review of a first breast may be easier to perform if the first breast is fatty, whereby the review may be performed by reading a first, smaller number of images; and a second diagnosis of a second breast may be more difficult if the second breast is dense, whereby the review may be performed by reading a second, greater number of images. The density of the breast may be determined by a Breast Imaging Reporting and Data System (BIRADS) classification, where BIRADS classifications A and B correspond to fattier, less dense breasts; a classification C corresponds to denser breasts; and classification D corresponds to breasts having a higher density than classification C.

In accordance with method 300 of FIG. 3, for example, a first case score may be assigned to images of breasts having a density within a BIRADS classifications A and B, resulting in the selection of a first reading protocol; a second case score may be assigned to images of breasts having a density within BIRADS classification C, resulting in the selection of a second reading protocol; and a third case score is assigned to images of breasts having a density within BIRADS classification D, resulting in the selection of a third reading protocol.

Method 300 of FIG. 3 may be applied to a theoretical (e.g., typical) patient population where 50% of the patients are classified in BIRADS classifications A and B, 40% of the patients are classified in BIRADS classification C, and 10% of the patients are classified in BIRADS classification D. The patients in BIRADS classifications A and B may be assigned a first case score indicating that a review of these patients may be performed by a radiologist using a protocol for reading an S2D image 1002, comprising four individual RMLO, LMLO, RCC, and LCC images. The patients in BIRADS classification C may be assigned a second case score indicating that a review of these patients may be performed by a radiologist using a protocol for reading S2D image 1002 and 16 slab images 1004, each slab image 1004 comprising four individual RMLO, LMLO, RCC, and LCC images, for a total of 68 images. The patients in BIRADS classification D may be assigned a third case score, indicating that a review of these patients may be performed by a radiologist using a protocol for reading S2D image 1002 and 50 slice images 1006, each slice image 1006 comprising four individual RMLO, LMLO, RCC, and LCC images, for a total of 204 images.

The total number of images included in each reading protocol may then be weighted by the percentage of patients in each classification, to calculate a relative reduction in images transferred to an RWS entailed by using the reading protocols described above rather than a traditional reading protocol described in reference to FIG. 9. For the BIRADS A and B classifications, a first weighted total number of images to be sent amounts to 4 * 0.5 (e.g., 50%) = 2 images. For the BIRADS C classification, a second weighted total number of images to be sent amounts to 68 * 0.4 (e.g., 40%) = 27.2 images. For the BIRADS D classification, a third weighted total number of images to be sent amounts to 204 * 0.1 (e.g., 10%) = 20.4 images. Summing the first, second, and third weighted total numbers of images results in a grand weighted total of nearly 50 images to be sent to the RWS to be read (e.g., 2 + 27.2 + 20.4 = 49.6).

From this it may be deduced that for an average patient case, where the patient has a 50% *a priori* probability of having a BIRADS A or B classification, a 40% *a priori* probability of having a BIRADS C classification, and a 10% *a priori* probability of having a BIRADS D classification, an average number of images specified by a reading protocol generated by the protocol generation system as described herein to be sent to the RWS may be 50. In comparison to the 268 images typically sent as described in reference to FIG. 9, the use of the protocol generation system may result in an 80% reduction in the amount of image data typically transferred over a network to be read by a radiologist. A similar benefit may be observed when the case scores and reading protocols are generated based on a cancer diagnosis made by an AI model (e.g., of anomaly detection systems 810 of FIG. 5), assuming statistical/historical cancer rates within a given patient population.

Thus, a protocol generation system is disclosed herein that performs an assessment of DBT image data, and selectively provides a minimum dataset and a reading protocol based on a score generated from the assessment. For some cases, an S2D-only minimum dataset and protocol may be sufficient. In other cases, an S2D + Slabs/Planes protocols may be provided, where there is less confidence in automated diagnoses. By sending an appropriate amount of image data to review based on the score, an amount of time spent by a radiologist reading DBT and synthetic 2D images may be reduced, without a decrease in a quality of the review. By reducing the amount of time spent reading the images, an amount of computational and memory resources of a computing system used to read the images may be reduced. Additionally, an amount of bandwidth consumed in sending images to the radiologist may be advantageously reduced, which may be used for other imaging tasks.

The technical effect of assessing DBT image data and selectively determining a minimum dataset of images to be reviewed based on a score generated from the assessment, is that an amount of computational and memory resources of a computing system used to read the images may be reduced, and an amount of bandwidth consumed in sending images to the radiologist may be reduced.

The disclosure also provides support for a protocol generation system for a digital mammography system, the protocol generation system comprising: a processor, and a memory storing instructions that when executed cause the processor to: receive digital breast tomosynthesis (DBT) case images of a patient, the DBT case images including at least one projection set of images and/or image volume of a breast of the patient reconstructed from data acquired using the digital mammography system, generate a case score for the DBT case images by analyzing the case images, select a minimum dataset of the DBT case images to be read, based on the case score, and send the minimum dataset and/or a reading protocol specifying the minimum dataset to a radiology workstation (RWS) to be read and/or store the minimum dataset in an archive. In a first example of the system, the protocol generation system is integrated into or electronically coupled to the digital mammography system or installed at a radiology workstation (RWS) communicatively coupled to the digital mammography system via a network. In a second example of the system, optionally including the first example, the received DBT case images are analyzed by one or more artificial intelligence (AI)-based anomaly detection systems, and the case score is generated by a protocol generation model of the protocol generation system, the protocol generation model taking as input an output of the one or more AI-based anomaly detection systems. In some embodiments, the case score may instead be generated using outputs from the one or more AI models. In some embodiments, the case score may instead be received as an input by an outside source. In a third example of the system, optionally including one or both of the first and second examples, the one or more AI-based anomaly detection systems include one of: a risk assessment system, a breast density assessment system, an anomaly detection system used to generate a set of slabs, a set of planes, and/or a Synthetic2D (S2D) image from the received DBT case images, and a different anomaly detection system. In a fourth example of the system, optionally including one or more or each of the first through third examples, the output of the anomaly detection system used to generate the set of slabs and/or the S2D image includes at least one of: a location of an anomaly in a received case image, a classification of the anomaly, an assessment of a seriousness of the anomaly, and a confidence score indicating a degree of confidence of the anomaly detection system in assessing the seriousness of the anomaly. In a fifth example of the system, optionally including one or more or each of the first through fourth examples, further instructions are stored in the memory that when executed, cause the processor to: in response to the case score being less than a first threshold value, not select a minimum dataset, in response to the case score being less than a second threshold value, select a minimum dataset including the S2D image and not including the set of slab images and the set of plane images, in response to the case score being less than a third threshold value, select a minimum dataset including the S2D image and the set of slab images and not including the set of plane images, and in response to the case score being greater than the third threshold value, select a minimum dataset including the S2D image, the set of slab images, and the set of plane images. In a sixth example of the system, optionally including one or more or each of the first through fifth examples, a full-field digital mammography (FFDM) image of the breast is an input into the protocol generation model, and the case score is generated at least partially based on the FFDM image. In a seventh example of the system, optionally including one or more or each of the first through sixth examples, further instructions are stored in the memory that when executed, depending on a classification of the FFDM image by an anomaly detection system, cause the processor to: in response to the case score being less than a first threshold value, not select a minimum dataset, in response to the case score being less than a second threshold value, select a minimum dataset including the FFDM image and not including the set of slab images and the set of plane images, in response to the case score being less than a third threshold value, select a minimum dataset including the FFDM image and the set of slab images and not including the set of plane images, and in response to the case score being greater than the third threshold value, select a minimum dataset including the FFDM image, the set of slab images, and the set of plane images. In a eighth example of the system, optionally including one or more or each of the first through seventh examples, the minimum dataset and/or an output of an anomaly detection system is encoded into a Digital Imaging and Communications in Medicine (DICOM) tag of the S2D image. In a ninth example of the system, optionally including one or more or each of the first through eighth examples, further instructions are stored in the memory that when executed, cause the processor to: compress the minimum dataset using a lossless compression, compress a remaining portion of the case images not included in the minimum dataset using lossy compression, and send both of the minimum dataset and the remaining portion to the archive.

The disclosure also provides support for a method, comprising: receiving digital breast tomosynthesis (DBT) case images of a breast of a patient, the DBT case images including at least one projection set of images and/or image volume of the breast, analyzing the received DBT case images using one or more artificial intelligence (aI)-based anomaly detection systems, generating a synthetic two-dimensional (S2D) image, a set of slab images, and a set of plane images from the DBT case images and the one or more aI-based anomaly detection systems, assigning a case score to the DBT case images based on an output of the one or more aI-based anomaly detection systems, using an aI model, selecting a reading protocol for reading the DBT case images based on the assigned case score, the reading protocol including a minimum dataset of the S2D image, the set of slab images, the set of plane images, and the DBT case images to be read, and sending the reading protocol and the minimum dataset to a radiology workstation (RWS) to be read and/or storing the minimum dataset in an archive. In a first example of the method, selecting the reading protocol for reading the DBT case images based on the assigned case score and sending the reading protocol and the minimum dataset to the RWS further comprises: in response to the case score being less than a first threshold value, not selecting the minimum dataset, in response to the case score being less than a second threshold value, selecting an S2D reading protocol where the minimum dataset includes the S2D image and does not include the set of slab images and the set of plane images, in response to the case score being less than a third threshold value, selecting an S2D+ slabs reading protocol where the minimum dataset includes the S2D image and the set of slab images and does not include the set of plane images, and in response to the case score being greater than the third threshold value, selecting an S2D+planes+slabs reading protocol where the minimum dataset includes all of the S2D image, the set of slab images, and the set of plane images. In a second example of the method, optionally including the first example, the method further comprises: encoding at least one of the reading protocol, the minimum dataset, and an output of an anomaly detection system into a Digital Imaging and Communications in Medicine (DICOM) tag of the S2D image. In a third example of the method, optionally including one or both of the first and second examples, the method further comprises: compressing the minimum dataset using a lossless compression, compressing a remaining portion of the DBT case images not included in the minimum dataset using lossy compression, and sending both of the minimum dataset and the remaining portion to the archive. In a fourth example of the method, optionally including one or more or each of the first through third examples, the method further comprises: receiving a full-field digital mammography (FFDM) image of the breast of the patient, assigning the case score to the DBT case images based on an output of the one or more AI-based anomaly detection systems and data from the FFDM image, using the AI model, consolidating data from the FFDM in the S2D image, including the FFDM image in the minimum dataset, and prioritizing a reading of the FFDM image in the selected reading protocol. In a fifth example of the method, optionally including one or more or each of the first through fourth examples, the AI model takes as input the output of the one or more AI-based anomaly detection systems and the DBT case images. In a sixth example of the method, optionally including one or more or each of the first through fifth examples, the AI model takes, as an additional input, an output of an anomaly detection system, the output including at least one of: a location of an anomaly in a received DBT case image, a classification of the anomaly, an assessment of a seriousness of the anomaly, and a confidence score indicating a degree of confidence of the anomaly detection system in assessing the seriousness of the anomaly.

The disclosure also provides support for a method, comprising: receiving, from a digital mammography system, a digital breast tomosynthesis (DBT) study of a breast of a patient comprising at least one of a synthetic two-dimensional (S2D) image, a set of slab images, a set of plane images, and an image volume, analyzing the DBT study using one or more artificial intelligence (AI)- based anomaly detection systems, assigning a case score to the DBT study based on an output of the one or more AI-based anomaly detection systems, using an AI model, selecting a reading protocol for reading the DBT study based on the assigned case score, the reading protocol including a minimum dataset of the DBT study to be read, and displaying the minimum dataset on a display device in accordance with the reading protocol. In a first example of the method, the reading protocol is extracted from a Digital Imaging and Communications in Medicine (DICOM) tag of the S2D image. In a second example of the method, optionally including the first example, the minimum dataset specified in the extracted reading protocol is retrieved from an archive of the digital mammography system at a first time, and remaining images of the DBT study not included in the minimum dataset is retrieved at a second time, the second time being later than the first time.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A protocol generation system for a digital mammography system, the protocol generation system comprising:
a processor, and a memory storing instructions that when executed cause the processor to:
receive digital breast tomosynthesis (DBT) case images of a patient, the DBT case images including at least one projection set of images and/or image volume of a breast of the patient reconstructed from data acquired using the digital mammography system;
generate or receive as an input a case score for the DBT case images;
select a minimum dataset of the DBT case images to be read, based on the case score; and
send the minimum dataset and/or a full dataset with a reading protocol specifying the minimum dataset to a radiology workstation (RWS) to be read and/or store the minimum dataset in an archive.

2. The protocol generation system of claim 1, wherein the protocol generation system is integrated into or electronically coupled to the digital mammography system or installed at a radiology workstation (RWS) communicatively coupled to the digital mammography system via a network.

3. The protocol generation system of claim 1, wherein the received DBT case images are analyzed by one or more artificial intelligence (AI) models, and the case score is generated using outputs from the one or more AI models.

4. The protocol generation system of claim 3, wherein the one or more AI models include one of:
a risk assessment system;
a negative triage system;
a breast density assessment system;
an anomaly detection system used to generate a set of planes, a set of slabs, and/or a Synthetic2D (S2D) image from the received DBT case images; and
a different anomaly detection system.

5. The protocol generation system of claim 4, wherein the output of the AI model is used to generate the set of planes, slabs and/or the S2D image includes at least one of:
a location of an anomaly in a received case image;
a classification of the anomaly;
an assessment of a seriousness of the anomaly; and
a confidence score indicating a degree of confidence of the anomaly detection system in assessing the seriousness or presence of the anomaly.

6. The protocol generation system of claim 1, wherein further instructions are stored in the memory that when executed, cause the processor to:
in response to the case score being in a first range of values, not select a minimum dataset;
in response to the case score being in a second range of values, select a minimum dataset including the S2D image and not including the set of slab images and the set of plane images;
in response to the case score being in a third range of values, select a minimum dataset including the S2D image and the set of slab images and not including the set of plane images; and
in response to the case score being in a fourth range of values, select a minimum dataset including the S2D image, the set of slab images, and the set of plane images.

7. The protocol generation system of claim 3, wherein a full-field digital mammography (FFDM) image of the breast is an input to the AI models, and the case score is generated at least partially based on the FFDM image.

8. The protocol generation system of claim 7, wherein further instructions are stored in the memory that when executed, depending on a classification of the FFDM image by an anomaly detection system, cause the processor to:
in response to the case score being in a first range of values, not select a minimum dataset;
in response to the case score being in a second range of values, select a minimum dataset including the FFDM image and not including the set of slab images and the set of plane images;
in response to the case score being in a third range of values, select a minimum dataset including the FFDM image and the set of slab images and not including the set of plane images; and
in response to the case score being in a fourth range of values, select a minimum dataset including the FFDM image, the set of slab images, and the set of plane images.

9. The protocol generation system of claim 5, wherein the reading protocol and/or an output of an anomaly detection system is encoded into a Digital Imaging and Communications in Medicine (DICOM) tag of the S2D image.

10. The protocol generation system of claim 1, wherein further instructions are stored in the memory that when executed, cause the processor to:
compress the minimum dataset using a lossless compression;
compress a remaining portion of the case images not included in the minimum dataset using lossy compression; and
send both of the minimum dataset and the remaining portion to the archive.

11. A method, comprising:
receiving digital breast tomosynthesis (DBT) case images of a breast of a patient, the DBT case images including at least one projection set of images and/or image volume of the breast;
analyzing the received DBT case images using one or more artificial intelligence (AI)- based anomaly detection systems;
generating a synthetic two-dimensional (S2D) image, a set of slab images, or a set of plane images from the DBT case images using the one or more AI-based anomaly detection systems;
assigning a case score to the DBT case images based on an output of the one or more AI-based anomaly detection systems,;
selecting a minimum dataset and a reading protocol for reading the DBT case images based on the assigned case score, the reading protocol including at least a reference to the images of the minimum dataset; and
sending the minimum dataset and/or the full dataset with the reading protocol to a radiology workstation (RWS) to be read and/or storing the minimum dataset in an archive.

12. The method of claim 11, wherein selecting the reading protocol for reading the DBT case images based on the assigned case score and sending the reading protocol and the minimum dataset to the RWS further comprises:
in response to the case score being in a first range of values, not selecting the minimum dataset;
in response to the case score being in a second range of values, selecting an S2D reading protocol where the minimum dataset includes the S2D image and does not include the set of slab images and the set of plane images;
in response to the case score being in a third range of values, selecting an S2D + slabs reading protocol where the minimum dataset includes the S2D image and the set of slab images and does not include the set of plane images; and
in response to the case score being in a fourth range of values, selecting an S2D+planes+slabs reading protocol where the minimum dataset includes all of the S2D image, the set of slab images, and the set of plane images.

13. The method of claim 11, further comprising encoding at least one of the reading protocol, and an output of an anomaly detection system into a Digital Imaging and Communications in Medicine (DICOM) tag of the S2D image.

14. The method of claim 12, further comprising:
compressing the minimum dataset using a lossless compression;
compressing a remaining portion of the DBT case images not included in the minimum dataset using lossy compression; and
sending both of the minimum dataset and the remaining portion to the archive.

15. A method, comprising:
receiving, from a digital mammography system, a digital breast tomosynthesis (DBT) study of a breast of a patient comprising at least one of a synthetic two-dimensional (S2D) image, a set of slab images, a set of plane images, and an image volume;
analyzing the DBT study using one or more artificial intelligence (AI)- based anomaly detection systems;
assigning a case score to the DBT study based on an output of the one or more AI-based anomaly detection systems, using an AI model;
selecting a reading protocol for reading the DBT study based on the assigned case score, the reading protocol including at least a reference to the images of the minimum dataset to be read; and
displaying the minimum dataset on a display device in accordance with the reading protocol.
